# EUROPÄISCHE PATENTSCHRIFT

(11) **EP 0 979 634 B1**
(45) Veröffentlichungstag und Bekanntmachung des Hinweises auf die Patenterteilung: **09.04.2003**
(21) Anmeldenummer: 99111075.0
(22) Anmeldetag: 17.06.1999
(51) Int. Cl.: A61B 3/15, A61B 3/117

(54) **Vorrichtung zur konfokalen Messung der Lichtreflexion eines Bereichs eines Körpers**
Device for the confocal measurement of light reflection of a body part
Dispositif pour la mesure confocale de la réflection de la lumière d'une partie d'un corps

(30) Priorität: 12.08.1998 DE 19836601
(43) Veröffentlichungstag der Anmeldung: 16.02.2000
(73) Patentinhaber: GSF-Forschungszentrum für Umwelt und Gesundheit GmbH, 85764 Oberschleissheim (DE)
(72) Erfinder: Frömel, Martin, 82343 Maising (DE); Kellerer, Albrecht, Prof., 80331 München (DE); Roos, Hartmut, Dr., 81245 München (DE)
(74) Vertreter: Reinhard - Skuhra - Weise & Partner

(56) Entgegenhaltungen:
- EP-A- 0 724 858
- US-A- 5 212 505
- US-A- 5 596 377
- MASSIG ET AL.: "Real-time confocal laser scan microscope for examination and diagnosis of the eye in vivo" APPLIED OPTICS, Bd. 33, Nr. 4, 1994, Seiten 690-694, XP002122434

## Beschreibung

Die vorliegende Erfindung betrifft eine Vorrichtung zur konfokalen Messung der Lichtreflexion eines Bereichs innerhalb eines transparenten Körpers, wie aus Massig et al.: "Real-time confocal laser scan microscope for examination and diagnosis of the eye *in vivo",* Applied Optics, Bd. 33, Nr. 4, 1994, S. 690-694 bekannt.

Aus der US-A-5,212,505 ist eine Einrichtung zum Richten eines Lichtstrahls auf einen Orientierungspunkt auf der Kornea und zum Erfassen des reflektierten Anteils des Lichtstrahls sowie eine Einrichtung zum Ableiten eines Signals für die gegenseitige Orientierung der optischen Achse und der Messachse bekannt. Gemäß dieser Druckschrift erfolgt die Ableitung des Orientierungssignals anhand des erfassten reflektierten Anteils und einer gemäß der geometrischen Gestalt der Kornea vorbestimmten Reflexionsbeziehung.

Die EP 0 724 858 A1 offenbart eine ophthalmologische Messvorrichtung mit einer Vorrichtung zur Bestimmung der Trübung, welche eine Messung in Form eines Tiefenscans durchführen kann.

Obwohl prinzipiell auf beliebige Körper anwendbar, werden die vorliegende Erfindung sowie die ihr zugrundeliegende Problematik in bezug auf konfokale Messungen der Lichtreflexion der Augenlinse eines Auges zur Bestimmung deren Trübung erläutert.

Bei vielen Fragestellungen der ophthalmologischen und epidemiologischen Forschung sowie in der ophthalmologischklinischen Praxis ist es notwendig, den Grad der Trübung der vorderen Augenabschnitte eines Patienten oder Probanden, insbesondere der Augenlinse, zu messen.

Eine Trübung tritt meistens nicht gleichmäßig über die gesamte Augenlinse auf, sondern in örtlich begrenzten Bereichen. Die menschliche Augenlinse weist eine zwiebelähnliche Schichtstruktur auf, und deshalb kommt es oft zu Trübungen innerhalb eines in Richtung der optischen Achse des Auges eng begrenzten Areals, das einer anatomischen Struktur entspricht. Aus diesem Grund ist es wünschenswert, den Grad der Linsentrübung, in Abhängigkeit von der Tiefe, quantitativ zu erfassen. Ein besonderes Problem ist dabei die definierte Zuordnung eines Trübungsmeßwertes zu der jeweiligen Position innerhalb des vorderen Augenabschnitts.

Bei einer nach dem konfokalen Prinzip arbeitenden Vorrichtung zur Untersuchung des vorderen Augenabschnitts wird ein Meßpunkt innerhalb des Auges mit einem Laserstrahl beleuchtet und der reflektierte und zurückgestreute Anteil von einem lichtempfindlichen Detektor registriert. Dabei wird das zu erfassende Licht durch eine kleine Lochblende fokussiert, und deswegen wird von einem dreidimensionalen Objekt nur die Zone wiedergegeben, die in der Brennebene des Objektivs liegt (optischer Schnitt). Um Informationen über Position, Dicke und Trübungszustand der anatomischen Strukturen des vorderen Augenabschnitts (Hornhaut, Vorderkammer, Linse) zu erhalten, muß die Brennebene des Objektivs innerhalb dieses Bereichs verschoben werden können.

Die der vorliegenden Erfindung zugrundeliegende Problematik besteht allgemein darin, daß für in vivo-Messungen dies so schnell erfolgen muß, daß die räumliche Zuordnung, d.h. die gegenseitige Orientierung des Auges und der Meßachse, nicht durch Augenbewegungen verfälscht wird.

Momentan finden sich im Stand der Technik folgende prinzipielle Ansätze für Meßgeräte für Messungen der Lichtreflexion der Augenlinse eines Auges zur Bestimmung deren Trübung. Eine Anordnung zur Untersuchung der vorderen Augenabschnitte mit Hilfe eines auf dem Scheimpflugprinzip basierenden Spaltlampengerätes ist beschrieben in Dragomirescu V., Hockwin O., Koch H.R., Sasaki K., "Development of a new equipment for rotating slit image photography according to Scheimpflug's principle", Interdiscipl. Topics Geront. 13 (1978), Seiten 118-130. Bei diesem bekannten Gerät wird eine mit einem Spaltlicht beleuchtete Schnittebene innerhalb des Auges in einer zur Beleuchtungsebene gekippten Aufnahmerichtung fotografiert. Die Auswertung der Aufnahmen erfolgt mikrodensitometrisch.

Die zeitraubende mikrodensitometrische Auswertung kann vermieden werden, indem die Bildregistrierung auf elektronischem Wege erfolgt. Eine derartige Vorrichtung ist in der DE 31 50 124 C2 beschrieben.

Ein Gerät zur Bestimmung der Gesamttrübung der Augenlinse ist das Lens Opacity Meter 701 der Firma Interzeag. Hier wird ein ausgedehnter Lichtstrahl ins Auge gesandt und der in einen bestimmten Winkelbereich gestreute Anteil gemessen. Diese Anordnung ist beispielsweise beschrieben in: Flammer J., Bebie H., Lens Opacity Meter: "A new instrument to quantify lens opacity", Ophthalmologica 195 (1987), Seite 69-72.

Konfokale Vorrichtungen werden in der Ophthalmologie hauptsächlich zur Untersuchung des Augenhintergrundes verwendet. Ein Gerät, bei dem die Brennebene derart verschoben werden kann, daß horizontale Schnittbilder von Hornhaut und Linse aufgenommen werden können, ist vorgestellt in: Massig J.H., Preissler M., Wegener A.R., Gaida G., "Real-time confocal laser scan microscope for examination and diagnosis of the eye in vivo", Applied Optics 33 (1994) Seiten 690-694.

Während der letzten Jahre sind darüberhinaus auf interferometrischen Verfahren basierende Geräte vorgestellt worden, mit denen intraokulare Distanzen wie Hornhaut- und Linsendicke, gemessen werden können. Davon können gegenwärtig nur die partielle Kohärenzinterferometrie und die Spektralinterferometrie für Messungen in vivo verwendet werden. Diese beiden Verfahren sind beispielsweise beschrieben in: Hitzenberger C.K., Drexler W., Baumgartner A., Lexer F., Sattmann H., Eselinger M., Kulhavy M., Fercher A.F., "Optical measurement of intraocular distances: A comparison of methods", Lasers and Light in Ophthalmology 8-2 (1997) Seiten 85-95.

Bei Untersuchungen der Augenlinse mit einer nach dem Scheimpflugprinzip arbeitenden Spaltlampenvorrichtung ist es notwendig, die Pupillen zu erweitern, da es sonst zu massiven Abschattungen kommt. Aber auch die erweiterte Pupille verursacht Abschattungen, die zu einer Verfälschung der gemessenen Trübungswerte führen. Ein besonderes Problem bei der Auswertung von Scheimpflugaufnahmen, insbesondere bei Verwendung von sichtbarem Licht, stellt die Lichtabschwächung innerhalb der Linse dar, die dazu führt, daß die gemessenen Trübungswerte im hinteren Bereich der Linse zu gering erscheinen. Da bei Scheimpfluganordnungen die Aufnahmerichtung gegenüber der optischen Achse des Auges geneigt ist, ergeben sich stets Probleme durch Abschattungen durch Stirn, Nase oder Lider.

Das o.e. Lens Opacity Meter integriert über Trübungen aller Hornhaut- und Linsenbereiche und liefert somit einen Wert, der proportional der Gesamttrübung des vorderen Augenabschnitts ist. Dieser Wert erlaubt keine Lokalisation einer Trübung und gibt keinen Aufschluß über biometrische Meßgrößen wie z.B. Lage oder Dicke von Hornhaut und Linse.

Bisher übliche konfokale Anordnungen für ophthalmologische Untersuchungen sind sehr aufwendig und kompliziert in Bedienung und Wartung. Sie sind für eine Bildebene parallel zur Pupillenebene ausgelegt. Um die tiefenabhängige Trübung von Hornhaut und Linse zu bestimmen, muß ein sehr hoher Aufwand betrieben werden, indem in Form eines Rasters zahlreiche Bilder aus verschiedenen Tiefen aufgenommen werden. Dies ist außerdem nicht so schnell zu bewerkstelligen, daß eine Beeinflussung des Ergebnisses durch Augenbewegungen vermieden werden könnte.

Daher ist es Aufgabe der vorliegenden Erfindung, eine verbesserte Vorrichtung zur konfokalen Messung der Lichtreflexion eines Bereichs eines Körpers, insbesondere der Augenlinse eines Auges zur Bestimmung deren Trübung, der eingangs erwähnten Art zu schaffen, bei der gegenseitige Orientierung von Meßeinrichtung und Körper genau bestimmbar sind.

Erfindungsgemäß wird diese Aufgabe durch die in Anspruch 1 angegebene Vorrichtung gelöst.

Die erfindungsgemäße Vorrichtung weist gegenüber den bekannten Lösungsansätzen den Vorteil auf, daß bei der Messung, z.B. von Linsentrübungen, die Beobachtungsrichtung mit der optischen Achse des Körpers bzw. Auges zusammenfallend gewählt werden kann, woraus sich erheblich weniger Abschattungsprobleme durch Pupille, Nase Stirn und Lidern ergeben als bei Untersuchungen nach dem Scheimpflugprinzip.

Die Vorrichtung erlaubt die Bestimmung und Lokalisation von Linsentrübungen sowie die Gewinnung von Meßdaten bezüglich Hornhautdicke, Vorderkammertiefe und Dicke der einzelnen Linsenstrukturen unter personal-, kosten- und zeitsparender Auswertung. Es vereinigt somit Vorteile anderer Vorrichtungen unterschiedlicher Prinzipien.

Die der vorliegenden Erfindung zugrundeliegende Idee besteht darin, daß eine Einrichtung zum Richten mindestens eines zweiten und dritten Lichtstrahls auf einen Orientierungspunkt auf der Oberfläche des Körpers und zum Erfassen des von dem Orientierungspunkt der Oberfläche des Körpers reflektierten Anteils des zweiten und dritten Lichtstrahls und eine Einrichtung zum Ableiten eines Signals für die gegenseitige Orientierung der optischen Symmetrieachse des Körpers und der Meßachse anhand des erfaßten, von dem Orientierungspunkt auf der Oberfläche des Körpers reflektierten Anteils des zweiten und dritten Lichtstrahls und einer gemäß der geometrischen Gestalt des Körpers vorbestimmten Reflexionsbeziehung vorgesehen sind.

In den Unteransprüchen finden sich vorteilhafte Weiterbildungen und Verbesserungen der in Anspruch 1 angegebenen Vorrichtung.

Gemäß einer bevorzugten Weiterbildung weist die Einrichtung zum Richten mindestens eines zweiten und dritten Lichtstrahls auf einen Orientierungspunkt eine ringförmige Halterung mit einer zur Meßachse parallelen Achse auf, wobei auf der zum Körper gerichteten Ringfläche zwei gegenüberliegende Paare aus einer jeweiligen Leuchtdiode und einem jeweiligen Photodiode derart angeordnet sind, daß der Lichtstrahl der jeweiligen Leuchtdiode über eine Reflexion durch den Körper auf die jeweiligen Photodiode gerichtet wird. Solch eine Anordnung, bestehend aus je zwei vorzugsweise spaltähnlichen Lichtquellen und geeigneten Detektoren, erlaubt eine parallele Ausrichtung auf die optische Achse des Auges.

Gemäß einer weiteren bevorzugten Weiterbildung ist die Frontlinse der Einrichtung zum Richten des auf einen Meßpunkt in dem Bereich fokussierten ersten Lichtstrahls in der Ebene im Ringinnern der ringförmigen Halterung derart angeordnet, daß die Achse und die Meßachse parallel zueinander verschiebbar sind. Die Einrichtung zur Bestimmung der Orientierung kann so relativ zur Meßeinrichtung verschoben werden. Eine Messung kann deshalb entlang einer beliebigen Achse parallel zur optischen Achse des Auges erfolgen.

Gemäß einer weiteren bevorzugten Weiterbildung ist die Frontlinse der Einrichtung zum Richten des auf einen Meßpunkt in dem Bereich fokussierten ersten Lichtstrahls zur Durchführung eines Tiefenscans in Richtung der Meßachse hin- und herbewegbar. Beispielsweise ist eine mechanische Führung der Frontlinse vorgesehen, durch die es möglich wird, die Frontlinse über eine motorbetriebene Kurbelvorrichtung vor- und zurückzubewegen. Die jeweilige Position kann von einer Vorrichtung zur Längenmessung, beispielsweise einem magnetoresistiven Sensor, gemessen werden und elektronisch einer Meßposition zugeordnet werden.

Gemäß einer weiteren bevorzugten Weiterbildung ist eine veschiebbare Einspannvorrichtung für den Körper vorgesehen, um diesen parallel zur Meßachse zu verschieben.

Gemäß einer weiteren bevorzugten Weiterbildung ist die Einrichtung zum Ableiten eines Signals für die gegenseitige Orientierung der optischen Achse des Körpers und der Meßachse derart gestaltet, daß sie ein Signal ausgibt, wenn die optische Achse und die Meßachse parallel zueinander sind, wobei das Signal die Einrichtung zum Richten eines auf einen Meßpunkt in dem Bereich fokussierten ersten Lichtstrahls entlang einer Meßachse und zum Erfassen des von dem Meßpunkt in dem Bereich entlang der Meßachse reflektierten Anteils des ersten Lichtstrahls aktiviert.

Ausführungsbeispiele der Erfindung sind in den Zeichnungen dargestellt und in der nachfolgenden Beschreibung näher erläutert.

Es zeigen:
- Fig. 1: eine schematische Darstellung einer Ausführungsform der erfindungsgemäßen Vorrichtung zur konfokalen Messung der Lichtreflexion der Augenlinse eines Auges zur Bestimmung deren Trübung;
- Fig. 2: einen Schnitt durch die Ebene der Frontlinse 4 in der Ringhalterung 40 in Fig. 1 in einer ersten Meßstellung; und
- Fig. 3: einen Schnitt durch die Ebene der Frontlinse 4 in der Ringhalterung 40 in Fig. 1 in einer zweiten Meßstellung.

In den Figuren bezeichnen gleiche Bezugszeichen gleiche oder funktionsgleiche Elemente.

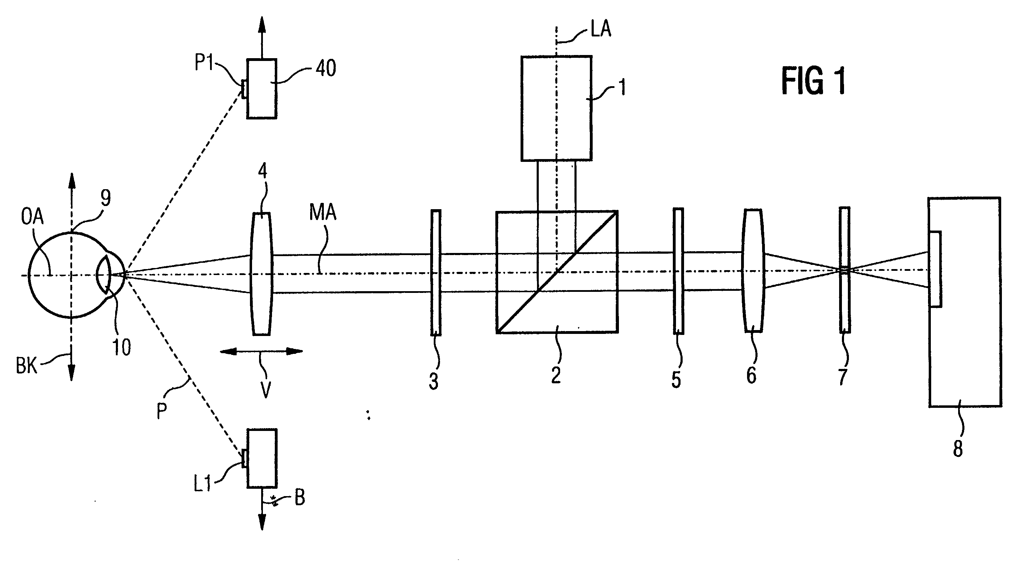

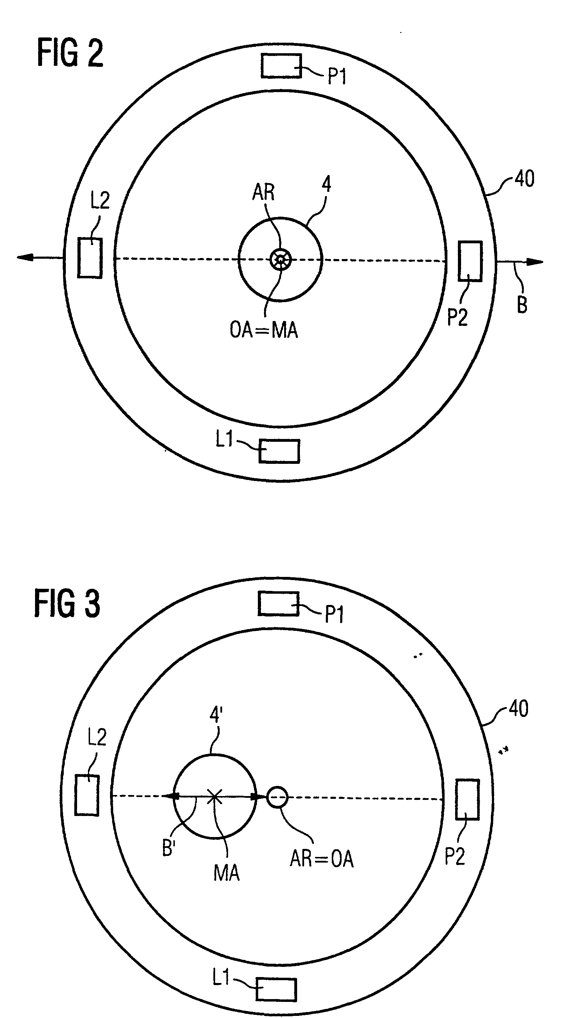

Fig. 1 zeigt eine schematische Darstellung einer Ausführungsform der erfindungsgemäßen Vorrichtung zur konfokalen Messung der Lichtreflexion der Augenlinse eines Auges zur Bestimmung deren Trübung und Fig. 2 einen Schnitt durch die Ebene der Frontline 4 in der Ringhalterung 40 in Fig. 1 in einer ersten Meßstellung.

Insbesondere bezeichnen 1 einen Laser mit einer Laserachse LA, 2 einen polarisierenden Strahlteiler, 3 ein Verzögerungsplättchen, 4 eine verschiebbare Frontlinse, V eine Verschiebungsrichtung der Frontlinse 4, 5 ein Interferenzfilter, 6 eine Objektivlinse, 7 eine Lochblende, 8 einen Detektor, 9 ein Auge, 10 eine Augenlinse, MA eine Meßachse, OA die optische Achse vom Auge 9, BK eine Bewegungsrichtung einer nicht gezeigten Kinnstütze, 40 eine Ringhalterung, L1, L2 Leuchtdioden, PT1, PT2 Photodioden, B, B' Relativbewegungsrichtungen von Ringhalterung/Frontlinse und AR eine Ringachse.

Der kollimierte Strahl des Lasers 1, z.B. in Form einer Laserdiode, wird im Strahlteiler 2 auf die Meßachse abgelenkt und nach Durchlaufen des Verzögerungsplättchens 3 von der Frontlinse 4 auf einen Punkt innerhalb der Augenlinse 10 fokussiert und von dieser zurückgestreut. Die Frontlinse 4 kollimiert das zurückgestreute Bündel zu einem Parallelstrahl, der dann nach Durchlaufen des Strahlteilers 2 und des Interferenzfilters 5 von der Objektivlinse 6 auf die Lochblende 7 fokussiert und von dem dahinter befindlichen lichtempfindlichen Detektor, beispielsweise einer Photomultiplierröhre, gemessen wird. Die Frontlinse wird durch eine mechanische Vorrichtung in Richtung V parallel zur Meßachse MA bewegt, um verschiedene Punkte in der Tiefe der Augenlinse 10 abzutasten. Die Position der Frontlinse wird dabei über ein Längenmeßsystem gemessen und dem jeweiligen Intensitätsmeßwert zugeordnet. Die Meßwerte werden digitalisiert und am PC ausgewertet.

Das wesentliche Merkmal besteht in der Art der Ermittlung der Ausrichtung von optischer Achse OA des Auges 9 und der Meßachse MA. Hierzu weist die Vorrichtung eine Einrichtung 40; P1, L1, P2, L2 zum Richten zweier Lichtstrahlen auf einen gemeinsamen Orientierungspunkt auf der Oberfläche des Auges 9 und zum Erfassen des von dem Orientierungspunkt der Oberfläche des Körpes reflektierten Anteils der beiden Lichtstrahlen auf. Fällt der Orientierungspunkt mit dem Durchtrittspunkt der optischen Achse zusammen, ist aufgrund der näherungsweise kugelsymmetrischen Gestalt die Reflexion maximal.

Die Einrichtung 40; P1, L1, P2, L2 weist bei dieser Ausführungsform eine ringförmige Halterung 40 mit einer zur Meßachse MA parallelen Achse AR auf. Auf der zum Auge 9 gerichteten Ringfläche sind zwei gegenüberliegende Paare aus einer jeweiligen Leuchtdiode L1, L2 und einem jeweiligen Photodiode P1, P2 derart angeordnet, daß der Lichtstrahl der jeweiligen Leuchtdiode L1, L2 über eine Reflexion durch das Auge 9 auf den jeweiligen Photodiode P1, P2 richtbar ist.

Um die Vorrichtung auf die optische Achse OA des Auges 9 auszurichten, wird also der spaltähnlich ausgeblendete Strahl der Leuchtdiode L1, L2, vorzugsweise Infrarotlicht, auf das Auge 9 gerichtet und der jeweilige reflektierte Strahl von der entsprechenden Photodiode P1, P2 detektiert. Die Ringhalterung 40 wird so lange gegenüber dem Auge 9 verfahren, bis das Signal der Photodioden P1, P2 maximal ist, wobei die Ringhalterung 40 gegenüber der bzw. senkrecht zur Meßachse MA verschoben werden kann. Im in Fig. 1 und 2 gezeigten Fall fallen optische Achse OA, Ringachse AR und Meßachse MA zusammen. Im in Fig. 3 gezeigten Fall fallen Ringachse AR und optische Achse OA zusammen, wobei die Meßachse MA zur optischen Achse OA verschoben ist, aber immer noch parallel zu dieser verläuft.

Prinzipiell kann die Messung also an einer beliebigen Stelle der Pupille parallel zur optischen Achse OA erfolgen.

Zur konfokalen Messung der Lichtreflexion bzw. Lichtstreuung eines Bereichs der Augenlinse eines Auges zur Bestimmung deren Trübung unter Verwendung der obigen Vorrichtung erfolgt also zunächst ein Anbringen des Kopfes eines Patienten in einer verstellbaren Kinnstütze. Dann erfolgt ein Ausrichten der Meßachse auf einen Meßpunkt der Augenlinse, und zwar entweder auf den Durchtrittspunkt der optischen Achse oder einen davon beabstandeten Punkt.

Dann erfolgt Verschieben der Einrichtung 40; P1, L1, P2, L2 parallel zur Meßachse, bis der reflektierte Anteil beider Leuchtdioden L1, L2 auf den Photodioden P1, P2 maximal ist.

Die Einrichtung zum Ableiten eines Signals für die gegenseitige Orientierung der optischen Achse OA des Auges 9 und der Meßachse MA gibt ein Signal aus, wenn die optische Achse OA und die Meßachse MA parallel zueinander sind.

Das Signal aktiviert die Einrichtung 1-8 zum Richten eines auf einen Meßpunkt in dem Bereich 10 fokussierten ersten Lichtstrahls entlang einer Meßachse MA und zum Erfassen des von dem Meßpunkt in dem Bereich 10 entlang der Meßachse MA reflektierten Anteils des ersten Lichtstrahls. Ist dies geschehen, erfolgt ein Durchführen der Messung in Form eines Tiefenscans. Die Frontlinse 4 der Einrichtung 1-8 zum Richten des auf einen Meßpunkt fokussierten ersten Lichtstrahls ist zur Durchführung des Tiefenscans in Richtung der Meßachse MA hin- und herbewegbar. Beispielsweise ist eine mechanische Führung der Objektivlinse vorgesehen, durch die es möglich wird, die Objektivlinse über eine motorbetriebene Kurbelvorrichtung vor- und zurückzubewegen. Die jeweilige Position kann von einer Vorrichtung zur Längenmessung, beispielsweise einem magnetoresistiven Sensor, gemessen werden.

Mit anderen Worten wird für jeden Tiefenwert ein Reflexionsmeßwert registriert und diesem der zugehörige Positionswert der Tiefe und die zugehörigen Abstandskoordinaten zur optischen Achse OA zugeordnet.

Die Messung wird verworfen, wenn sich während der Messung der reflektierte Anteil der beiden Lichtstrahlen zur Orientierungsbestimmung ändert.

Soll an einem anderen Meßpunkt in Tiefenrichtung gemessen werden, so wird das Auge relativ zur Meßachse MA in Richtung BK verschoben bzw. die Kinnstütze verstellt.

Dann erfolgt in analoger Weise die Orientierungsermittlung und dann der Tiefenscan, beispielsweise für die in Fig. 3 gezeigte Orientierung.

Um vor der Untersuchung einen Überblick über die vorliegenden Linsentrübungen zu erhalten, ist die Kombination des konfokalen Gerätes mit einer Retroilluminationsanordnung vorgesehen.

Obwohl die vorliegende Erfindung vorstehend anhand bevorzugter Ausführungsbeispiele beschrieben wurde, ist sie darauf nicht beschränkt, sondern auf vielfältige Art und Weise modifizierbar.

Obwohl beim gezeigten Ausführungsbeispiel die Einrichtung zum Ableiten eines Signals für die gegenseitige Orientierung der optischen Achse des Körpers und der Meßachse anhand des erfaßten, von dem Orientierungspunkt auf der Oberfläche des Körpes reflektierten Anteils des zweiten Lichtstrahls und einer gemäß der geometrischen Gestalt des Körpers vorbestimmten Reflexionsbeziehung eine maximale Reflexion an einem Orientierungspunkt erfaßt, ist diese auch auf andere Weise realisierbar. Beispielsweise können Referenz-Reflexionswerte im voraus gespeichert werden und die Orientierung durch einen Vergleich damit ermittelt werden.

Auch sind beliebige Lichtquellen und Lichtdetektoren verwendbar.

Obwohl der Rahmen für die Einrichtung zum Richten mindestens eines zweiten Lichtstrahls auf einen Orientierungspunkt auf der Oberfläche des Körpers und zum Erfassen des ,von dem Orientierungspunkt der Oberfläche des Körpes reflektierten Anteils des zweiten Lichtstrahls oben als kreisringförmig dargestellt wurde, ist seine geometrische Ringform beliebig.

Auch ist nicht unbedingt ein Ring erforderlich, sondern es genügen Lichtquellen/Detektor-Paare mit bestimmter Orientierung zur Frontlinse bzw. Meßachse.

### BEZUGSZEICHENLISTE:

- 1: Laser
- 2: polarisierender Strahlteiler
- 3: Verzögerungsplättchen
- 4: verschiebbare Frontlinse
- V: Verschiebungsrichtung
- 5: Interverenzfilter
- 6: Objektivlinse
- 7: Lochblende
- 8: Detektor
- 9: Auge
- 10: Augenlinse
- LA: Laserachse
- MA: Meßachse
- OA: optische Achse vom Auge
- BK: Bewegungsrichtung Kinnstütze
- 40: Ringhalterung
- L1, L2: Leuchtdioden
- PT1, PT2: Photodiodeen
- B, B': Relativbewegungsrichtung Rinhalterung/ Frontlinse
- AR, AR': Ringachse

## Patentansprüche

1. Vorrichtung zur konfokalen Messung der Lichtreflexion eines Bereichs (10) innerhalb eines transparenten Körpers (9), insbesondere der Augenlinse eines Auges zur Bestimmung deren Trübung, wobei der Körper (9) eine optische Symmetrieachse (OA) aufweist, mit einer Einrichtung (1-8) zum Richten eines auf einen Meßpunkt in dem Bereich (10) fokussierten ersten Lichtstrahls entlang einer Meßachse (MA) und zum Erfassen des von dem Meßpunkt in dem Bereich (10) entlang der Meßachse (MA) reflektierten Anteils des ersten Lichtstrahls;
**gekennzeichnet durch**
eine Einrichtung (40; P1, L1, P2, L2) zum Richten mindestens eines zweiten und eines dritten Lichtstrahls auf einen Orientierungspunkt auf der Oberfläche des Körpers (9) und zum Erfassen des von dem Orientierungspunkt der Oberfläche des Körpes reflektierten Anteils des zweiten und dritten Lichtstrahls; und
eine Einrichtung zum Ableiten eines Signals für die gegenseitige Orientierung der optischen Symmetrieachse (OA) des Körpers (9) und der Meßachse (MA) anhand des erfaßten, von dem Orientierungspunkt auf der Oberfläche des Körpes (9) reflektierten Anteils des zweiten und dritten Lichtstrahls und einer gemäß der geometrischen Gestalt des Körpers (9) vorbestimmten Reflexionsbeziehung.

2. Vorrichtung nach Anspruch 1, **dadurch gekennzeichnet, daß** die Einrichtung (40; P1, L1, P2, L2) zum Richten mindestens eines zweiten und dritten Lichtstrahls auf einen Orientierungspunkt eine ringförmige Halterung (40) mit einer zur Meßachse (MA) parallelen Achse (AR, AR') aufweist, wobei auf der zum Körper (9) gerichteten Ringfläche zwei gegenüberliegende Paare aus einer jeweiligen Leuchtdiode (L1, L2) und einem jeweiligen Photodiode (P1, P2) derart angeordnet sind, daß der Lichtstrahl der jeweiligen Leuchtdiode (L1, L2) über eine Reflexion durch den Körper (9) auf die jeweilige Photodiode (P1, P2) gerichtet wird.

3. Vorrichtung nach Anspruch 1, **dadurch gekennzeichnet, daß** die Frontlinse (4) der Einrichtung (1-8) zum Richten des auf einen Meßpunkt in dem Bereich (10) fokussierten ersten Lichtstrahls in der Ebene im Ringinnern der ringförmigen Halterung (40) derart angeordnet ist, daß die Achse (AR, AR') und die Meßachse (MA) parallel zueinander verschiebbar sind.

4. Vorrichtung nach einem der vorhergehenden Ansprüche, **dadurch gekennzeichnet, daß** die Frontlinse (4) der Einrichtung (1-8) zum Richten des auf einen Meßpunkt in dem Bereich (10) fokussierten ersten Lichtstrahls zur Durchführung eines Tiefenscans in Richtung der Meßachse (MA) hinund herbewegbar ist.

5. Vorrichtung nach einem der vorhergehenden Ansprüche, **dadurch gekennzeichnet, daß** eine verschiebbare Einspannvorrichtung für den Körper (9) vorgesehen ist, um diesen parallel zur Meßachse (MA) zu verschieben.

6. Vorrichtung nach einem der vorhergehenden Ansprüche, **dadurch gekennzeichnet, daß** die Einrichtung zum Ableiten eines Signals für die gegenseitige Orientierung der optischen Symmetrieachse (OA) des Körpers (9) und der Meßachse (MA) ein Signal ausgibt, wenn die optische Symmetrieachse (OA) und die Meßachse (MA) parallel zueinander sind, wobei das Signal die Einrichtung (1-8) zum Richten eines auf einen Meßpunkt in dem Bereich (10) fokussierten ersten Lichtstrahls entlang einer Meßachse (MA) und zum Erfassen des von dem Meßpunkt in dem Bereich (10) entlang der Meßachse (MA) reflektierten Anteils des ersten Lichtstrahls aktiviert.

7. Verfahren zur konfokalen Messung der Lichtreflexion eines Bereichs der Augenlinse eines Auges zur Bestimmung deren Trübung unter Verwendung der Vorrichtung gemäß mindestens einem der vorhergehenden Ansprüche mit den Schritten:
Anbringen des Kopfes eines Patienten in einer verstellbaren Kinnstütze;
Ausrichten der Meßachse auf einen Meßpunkt der Augenlinse;
Verschieben der Einrichtung (40; P1, L1, P2, L2) zum Richten mindestens eines zweiten und dritten Lichtstrahls auf einen Orientierungspunkt auf der Oberfläche des Körpers (9) und zum Erfassen des von dem Orientierungspunkt der Oberfläche des Körpes reflektierten Anteils des zweiten und dritten Lichtstrahls parallel zur Meßachse, bis der reflektierte Anteil maximal ist; und
Durchführen der Messung in Form eines Tiefenscans.

8. Verfahren nach Anspruch 7, **dadurch gekennzeichnet, daß** die Messung verworfen wird, wenn sich während der Messung der reflektierte Anteil des zweiten und dritten Lichtstrahls ändert.

9. Verfahren nach Anspruch 8, **dadurch gekennzeichnet, daß** der Meßpunkt durch Verstellen der Ausrichtung zwischen Kinnstütze und Meßachse verändert wird.

## Claims

1. Apparatus for the confocal measurement of the reflection of light of a region (10) inside a transparent body (9), in particular the lens of an eye, in order to determine the opaqueness thereof, the body (9) having an optical axis of symmetry (OA), having a device (1-8) for directing a first light beam, which is focused onto a measuring point in the region (10), along a measurement axis (MA) and for detecting the portion of the first light beam that is reflected from the measuring point in the region (10) along the measurement axis (MA);
**characterised by**
a device (40; P1, L1, P2, L2) for directing at least a second and a third light beam onto an orientation point on the surface of the body (9) and for detecting the portions of the second and third light beams that are reflected from the orientation point of the surface of the body; and
a device for deriving a signal for the mutual orientation of the optical axis of symmetry (OA) of the body (9) and the measurement axis (MA) on the basis of the detected portions of the second and third light beams that are reflected from the orientation point on the surface of the body (9), and a reflection relationship which is predetermined according to the geometrical form of the body (9).

2. Apparatus according to claim 1, **characterised in that** the device (40; P1, L1, P2, L2) for directing at least a second and third light beam onto an orientation point has an annular holder (40) having an axis (AR, AR') which is parallel with the measurement axis (MA), two opposing pairs comprising a respective light-emitting diode (L1, L2) and a respective photodiode (P1, P2) being arranged on the annular face, which is directed towards the body (9), in such a manner that the light beam of the respective light-emitting diode (L1, L2) is directed onto the respective photodiode (P1, P2) via a reflection by the body (9).

3. Apparatus according to claim 1, **characterised in that** the front lens (4) of the device (1-8) for directing the first light beam, which is focused onto a measuring point in the region (10), is arranged in the plane within the annular holder (40) in such a manner that the axis (AR, AR') and the measurement axis (MA) can be displaced in parallel with each other.

4. Apparatus according to any one of the preceding claims, **characterised in that** the front lens (4) of the device (1-8) for directing the first light beam, which is focused onto a measuring point in the region (10), can be moved back and forth in the direction of the measurement axis (MA) in order to carry out a depth scan.

5. Apparatus according to any one of the preceding claims, **characterised in that** a displaceable clamping apparatus is provided for the body (9) in order to displace the body (9) parallel with the measurement axis (MA).

6. Apparatus according to any one of the preceding claims, **characterised in that** the device for deriving a signal for the mutual orientation of the optical axis of symmetry (OA) of the body (9) and the measurement axis (MA) emits a signal if the optical axis of symmetry (OA) and the measurement axis (MA) are parallel with each other, the signal activating the device (1-8) for directing a first light beam, which is focused onto a measuring point in the region (10), along a measurement axis (MA), and for detecting the portion of the first light beam that is reflected from the measuring point in the region (10) along the measurement axis (MA).

7. Method for the confocal measurement of the reflection of light of a region of the lens of an eye in order to determine the opaqueness thereof using the apparatus according to at least one of the preceding claims, comprising the steps:
positioning the head of a patient in an adjustable chin support;
alignment of the measurement axis relative to a measuring point of the eye lens;
displacement of the device (40; P1, L1, P2, L2) for directing at least a second and third light beam onto an orientation point on the surface of the body (9) and for detecting the portions of the second and third light beams that are reflected from the orientation point of the surface of the body, parallel with the measurement axis until the reflected portion is at a maximum; and
carrying out the measurement in the form of a depth scan.

8. Method according to claim 7, **characterised in that** the measurement is rejected if the reflected portions of the second and third light beam change during the measurement.

9. Method according to claim 8, **characterised in that** the measuring point is changed by adjusting the alignment between the chin support and the measurement axis.

## Revendications

1. Appareil pour la mesure confocale de la réflexion de la lumière d'une région (10) à l'intérieur d'un corps transparent (9), en particulier le cristallin d'un oeil, pour déterminer son opacité, dans lequel le corps (9) présente une symétrie optique (OA), avec un dispositif (1-8) pour diriger un premier rayon lumineux focalisé sur un point de mesure dans la région (10) le long d'un axe (MA) de mesure et pour recueillir la portion du premier rayon lumineux réfléchie dans la région (10) le long de l'axe (MA) de mesure ;
**caractérisé par**
un dispositif (40 ; P1, L1, P2, L2) pour diriger au moins un deuxième et un troisième rayons lumineux sur un point d'orientation sur la surface du corps (9) et pour recueillir la partie des deuxième et troisième rayons lumineux réfléchie par le point d'orientation sur la surface du corps ; et
un dispositif pour extraire un signal pour l'orientation mutuelle de l'axe optique (OA) de symétrie du corps (9) et de l'axe (MA) de mesure à partir de la partie recueillie des deuxième et troisième rayons lumineux réfléchie par le point d'orientation sur la surface du corps et d'une relation de réflexion prédéterminée en fonction de la structure géométrique du corps (9).

2. Appareil selon la revendication 1, **caractérisé en ce que** le dispositif (40 ; P1, L1, P2, L2) pour diriger au moins un deuxième et un troisième rayons lumineux sur un point d'orientation présente un raccord (40) en forme d'anneau avec un axe (AR, AR') parallèle à l'axe (MA) de mesure, dans lequel, sur la surface en forme d'anneau tournée vers le corps (9), deux paires composées respectivement d'une diode électroluminescente (L1, L2) et d'une photodiode (P1, P2) correspondante sont disposées en regard l'une de l'autre de sorte que le rayon lumineux de la diode électroluminescente (L1, L2) correspondante soit dirigé au moyen d'une réflexion par le corps (9) sur la photodiode (P1, P2) correspondante.

3. Appareil selon la revendication 1, **caractérisé en ce que** la lentille frontale (4) du dispositif (1-8) pour diriger un premier rayon lumineux focalisé sur un point de mesure dans la région (10) est disposée dans le plan à l'intérieur de l'anneau du raccord (40) en forme d'anneau de sorte que l'axe (AR, AR') et l'axe (MA) de mesure puissent glisser parallèlement l'un à l'autre.

4. Appareil selon l'une des revendications précédentes, **caractérisé en ce que** la lentille frontale (4) du dispositif (1-8) pour diriger un premier rayon lumineux focalisé sur un point de mesure dans la région (10) peut être déplacée en va-et-vient pour réaliser un balayage en profondeur dans la direction de l'axe (MA) de mesure.

5. Appareil selon l'une des revendications précédentes, **caractérisé en ce qu'**un dispositif coulissant de serrage est prévu pour le corps (9) afin de le déplacer parallèlement à l'axe (MA) de mesure.

6. Appareil selon l'une des revendications précédentes, **caractérisé en ce que** le dispositif pour extraire un signal pour l'orientation mutuelle de l'axe optique (OA) de symétrie du corps (9) et de l'axe (MA) de mesure émet un signal lorsque l'axe optique (OA) de symétrie et l'axe (MA) de mesure sont parallèles l'un à l'autre, dans lequel le signal active le dispositif (1-8) pour diriger un premier rayon lumineux focalisé sur un point de mesure dans la région (10) le long d'un axe (MA) de mesure et pour recueillir la portion du premier rayon lumineux réfléchie dans la région (10) le long de l'axe (MA) de mesure.

7. Procédé pour la mesure confocale de la réflexion de la lumière d'une région du cristallin d'un oeil pour déterminer son opacité, utilisant l'appareil selon au moins l'une des revendications précédentes, comprenant les étapes consistant à :
amener la tête d'un patient dans un support réglable de menton ;
diriger l'axe de mesure sur un point de mesure du cristallin ;
déplacer le dispositif (40 ; P1, L1, P2, L2) pour diriger au moins un deuxième et un troisième rayons lumineux sur un point d'orientation sur la surface du corps (9) et pour recueillir la partie des deuxième et troisième rayons lumineux réfléchie parallèlement à l'axe de mesure par le point d'orientation sur la surface du corps, jusqu'à ce que la partie réfléchie soit maximale ; et
effectuer la mesure sous la forme d'un balayage en profondeur.

8. Procédé selon la revendication 7, **caractérisé en ce que** la mesure est rejetée quand, pendant la mesure, la partie réfléchie des deuxième et troisième rayons lumineux varie.

9. Procédé selon la revendication 8, **caractérisé en ce que** le point de mesure est modifié en réglant l'orientation entre le support de menton et l'axe de mesure.
